# EUROPEAN PATENT APPLICATION

(11) **EP 3 040 953 A1**
(43) Date of publication of application: **06.07.2016**
(21) Application number: 15202536.7
(22) Date of filing: 23.12.2015
(51) Int. Cl.: G07F 17/00, G07F 11/62

(54) **DRUG DISPENSING SYSTEM AND METHOD FOR CONTROLLING THE SAME**

(30) Priority: 31.12.2014 KR 20140195096
(71) Applicant: JVM Co., Ltd., Daegu 42709 (KR)
(72) Inventor: KIM, Jun Ho, Daegu, 42093 (KR)
(74) Representative: dompatent von Kreisler Selting Werner - Partnerschaft von Patent- und Rechtsanwälten mbB

(57) **Abstract**

A drug dispensing system includes at least one drug dispensing device including a plurality of dispensing modules for receiving and dispensing drugs, a plurality of installation blocks for allowing the dispensing modules to be mounted therein, and a controller for controlling dispensing of drugs from the dispensing modules, a plurality of drug refilling stations, a drug refilling unit for performing a drug refilling operation, a refilling authentication device for authenticating refilling information regarding the drugs refilled by the drug refilling unit, and a communication unit for transmitting authentication information regarding the first dispensing module confirmed by the refilling authentication device, a server for receiving the authentication information regarding the first dispensing module from the communication unit, and a database for storing the authentication information regarding the first dispensing module received from the server.

## Description

### BACKGROUND OF THE INVENTION

### Field of the Invention

The present invention relates to a drug dispensing system and a method of controlling the same, and more particularly to a drug dispensing system configured such that only a dispensing module that has been authenticated by a drug refilling station assigned to the drug dispensing system in advance is controlled to perform a drug dispensing operation and a method of controlling the same.

### Description of the Related Art

In general, a dose of drugs based on a prescription provided to a patient may include various kinds and types of drugs, and doses of drugs sorted according to the prescription may be transmitted to the patient while being contained in a basket.

Drugs may be collected in a basket from boxes containing respective drugs based on the kind and number of drugs set forth in the prescription for the patient. The basket containing the drugs therein may be transmitted to the patient, and the patent may take the drugs collected in the basket.

Conventionally, in order to collect various kinds of drugs in a single basket, it is necessary for a drug expert, that is, a pharmacist, to manually take the drugs out from bottles containing drugs and prepare doses of drugs based on the prescription for the patient. For this reason, it is necessary to confirm whether the drugs have been correctly collected.

As a result, it is difficult to correctly administer drugs, whereby a medical incident may occur. Furthermore, the collection of drugs is complicated, and increases the time necessary to collect drugs based on the prescription for the patient, thereby reducing the efficiency with which drug are prepared.

For this reason, there is a high necessity for a method or technology that is capable of improving the correctness and efficiency with which each dose of drugs is collected based on the prescription for the patient, thereby improving the convenience of the user, that is, the pharmacist, and preventing the occurrence of a medical incident.

### SUMMARY OF THE INVENTION

Therefore, the present invention has been made in view of the above problems, and it is an object of the present invention to provide a drug dispensing system configured such that a drug refilling operation performed by a plurality of drug refilling stations is managed using a single client PC, thereby improving the efficiency of drug collection.

It is another object of the present invention to provide a drug dispensing system configured such that only a dispensing module that has been authenticated in advance by a drug refilling station assigned to the drug dispensing system is controlled to perform a drug dispensing operation.

In accordance with an aspect of the present invention, the above and other objects can be accomplished by the provision of a drug dispensing system including at least one drug dispensing device including a plurality of dispensing modules for receiving and dispensing drugs, a plurality of installation blocks for allowing the dispensing modules to be mounted therein, and a controller for controlling the dispensing of drugs from the dispensing modules, a plurality of drug refilling stations, each drug refilling station including an input unit for allowing information related to a first dispensing module that needs to be refilled with drugs, selected from among the dispensing modules, to be input, a drug refilling unit for performing a drug refilling operation necessary to refill the first dispensing module with corresponding drugs according to the information related to the first dispensing module, a refilling authentication device for authenticating refilling information regarding the drugs refilled by the drug refilling unit, and a communication unit for transmitting authentication information regarding the first dispensing module confirmed by the refilling authentication device, a server for receiving the authentication information regarding the first dispensing module from the communication unit, and a database for storing the authentication information regarding the first dispensing module received from the server, wherein, when a second dispensing module is mounted in the installation block, the controller requests authentication information regarding the second dispensing module from the server, and, upon determining that the authentication information regarding the second dispensing module does not exist in the database, controls a drug dispensing operation not to be performed.

The controller may include a stock management means for determining which of the dispensing modules needs to be refilled with drugs, and at least one dispensing module that has been determined to need to be refilled with drugs by the stock management means may be mounted in the drug refilling unit.

The information related to the dispensing module may include at least the name of drugs with which to refill the dispensing module, identification information of the dispensing module, and information regarding the quantity of drugs with which to refill the dispensing module.

The refilling authentication device may be a barcode reader for reading the refilling information. Alternatively, the refilling authentication device may be a radio frequency identification (RFID) reader for reading the refilling information.

The refilling authentication device may include a quantity measurement device for measuring the quantity of drugs with which the dispensing module has been refilled, and the quantity of drugs with which the dispensing module has been refilled may be estimated based on the weight of the drugs measured by the quantity measurement device.

The refilling information confirmed by the refilling authentication device may include at least identification information of the drugs with which the dispensing module has been refilled and information regarding the quantity of the drugs.

The authentication information may include at least the name of drugs with which the dispensing module has been refilled, identification information of the dispensing module, and information regarding the quantity of drugs with which the dispensing module has been refilled.

Upon determining that authentication information regarding the first dispensing module exists in the database, the controller may control the drug dispensing operation to be performed.

In accordance with another aspect of the present invention, there is provided a method of controlling a drug dispensing system, the method including mounting a dispensing module that has been refilled, transmitting a request of authentication information regarding the mounted dispensing module, receiving a response signal indicating whether authentication information regarding the mounted dispensing module exists in response to the request of the authentication information, and, when the response signal indicates that the authentication information regarding the refilled dispensing module exists, controlling the refilled dispensing module to perform a drug dispensing operation.

The method may further include, when the response signal indicates that no authentication information regarding the refilled dispensing module exists, controlling the refilled dispensing module so as not to perform the drug dispensing operation.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a view schematically illustrating an environment in which a drug dispensing system according to an embodiment of the present invention is operated;
FIG. 2 is a perspective view schematically showing a drug dispensing device according to an embodiment of the present invention;
FIG. 3 is a perspective view schematically showing an installation block and a dispensing module of FIG. 2;
FIG. 4 is a perspective view schematically showing an installation block and a dispensing module according to another embodiment of the present invention;
FIG. 5 is an exploded perspective view showing the dispensing module of FIG. 4;
FIG. 6 is a block diagram showing the construction of the drug dispensing device according to the embodiment of the present invention in detail;
FIG. 7 is a block diagram showing the construction of a drug refilling station according to an embodiment of the present invention in detail;
FIG. 8 is a flowchart illustrating a drug refilling method according to an embodiment of the present invention;
FIGS. 9 to 13 are views showing an example of a user interface for controlling the drug refilling station according to the embodiment of the present invention;
FIG. 14 is a flowchart illustrating a method of controlling the drug dispensing system according to the embodiment of the present invention; and
FIG. 15 is a table illustrating information stored in a database of the drug dispensing system according to the embodiment of the present invention.

### DETAILED DESCRIPTION OF THE INVENTION

Reference will now be made in detail to the preferred embodiments of the present invention, examples of which are illustrated in the accompanying drawings.

In the following description of the present invention, a detailed description of known functions and configurations incorporated herein will be omitted when it may obscure the subject matter of the present invention.

The terms used in this specification are provided only to explain the present invention. Therefore, the present invention is not limited by the terms used in this specification. In this specification, the term "connection" or "coupling" does not refer only to direct connection or coupling, but also refers to indirect connection or coupling via another component. In addition, the terms "module" and "unit" are used for convenience of description and thus can be used interchangeably and do not have any distinguishable meanings or functions.

In this specification, the terms "first," "second," etc. are used only to distinguish one element from another element.

Hereinafter, the present invention will be described in more detail with reference to the accompanying drawings. The drawings are provided only for understanding of the present invention, and therefore the technical idea of the present invention is not limited by the drawings. Meanwhile, the same components or parts are denoted by the same reference numerals, and a duplicated description may be omitted.

In the following description, "dispensing modules" and "installation blocks" will be defined as follows for the convenience of description. Each of the dispensing modules is a minimum unit component which includes a drug storage unit for storing a specific kind of drugs and which is configured such that the drugs can be dispensed from the dispensing module and the dispensing module can be refilled with drugs. Each of the installation blocks is a component which includes a plurality of mounting units provided in a drug dispensing device for allowing the dispensing modules to be mounted therein and which provides an interface for enabling the dispensing modules to be constituted and treated as a bundle in a spatial and/or control aspect. For example, each of the dispensing modules may be a drug storage box, a drug dispensing box, or a division block.

The present invention may be modified or varied without departing from the technical idea and scope of the present invention. Modifications and variations within the technical idea and scope of the present invention are obvious to those skilled in the art. Consequently, the present invention includes modifications and variations within the technical idea and scope of the present invention. In addition, the present invention is not limited by embodiments, which will be described hereinafter.

### 1. Operating Environment

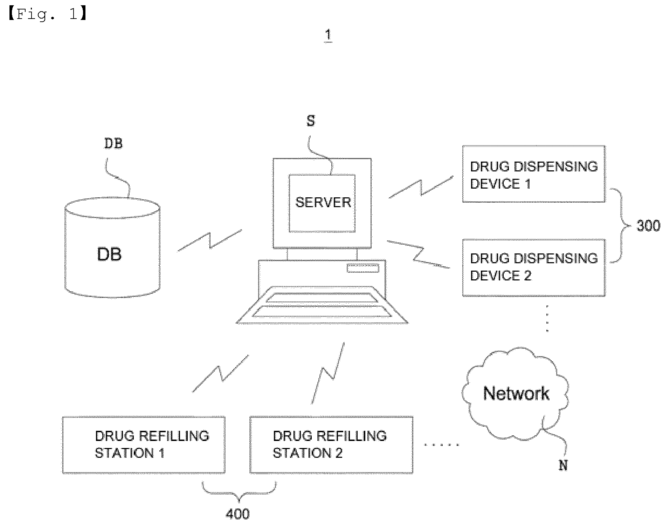

FIG. 1 is a view schematically illustrating an environment in which a drug dispensing system according to an embodiment of the present invention is operated.

Referring to FIG. 1, a drug dispensing system 1 according to an embodiment of the present invention may include at least one drug dispensing device 300, at least one drug refilling station 400, a server S, and a database DB. In this embodiment, the drug dispensing system 1 may include a plurality of drug dispensing devices 300 and/or a plurality of drug refilling stations 400.

Specifically, as shown in FIG. 1, the server S, the drug dispensing devices 300, and/or the drug refilling stations 400 may be connected to one another via a predetermined network N. The network N may include wired and wireless networks. For example, in a case in which the drug dispensing system 1 is disposed in a hospital, a plurality of drug refilling stations 400 and/or a plurality of drug dispensing devices 300 assigned to a server S installed in the hospital (hereinafter, referred to as a 'server') may be connected to the server via the network N.

The server S may receive various kinds of information regarding patients, identification information and/or authentication information regarding users, information related to dispensing modules that need to be refilled with drugs, and authentication information regarding dispensing modules that have been refilled with drugs from the drug dispensing devices 300 and/or the drug refilling stations 400.

For example, the various kinds of information regarding patients may include personal information, such as names, sexes, and ages of patients, information regarding clinical histories, such as disease histories, of patents, and information related to prescriptions regarding drugs that patients have to take. The identification information and/or authentication information regarding users, the information related to dispensing modules that need to be refilled with drugs, and the authentication information regarding dispensing modules that have been refilled with drugs will be described in detail with reference to related drawings.

The database DB may be connected to the server S via the network N. The database DB may store the various kinds of information regarding patients, the identification information and/or authentication information regarding users, information related to dispensing modules that need to be refilled with drugs, and authentication information regarding dispensing modules that have been refilled with drugs, received by the server S.

### 2. Construction of Drug Dispensing Device

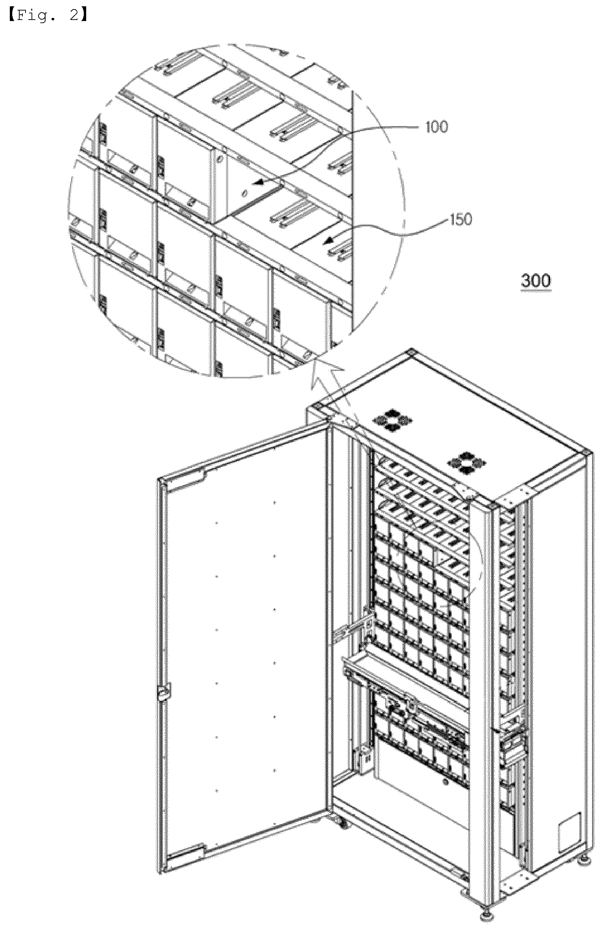

Hereinafter, the construction of a drug dispensing device according to an embodiment of the present invention will be described in detail with reference to FIGS. 2 to 6.

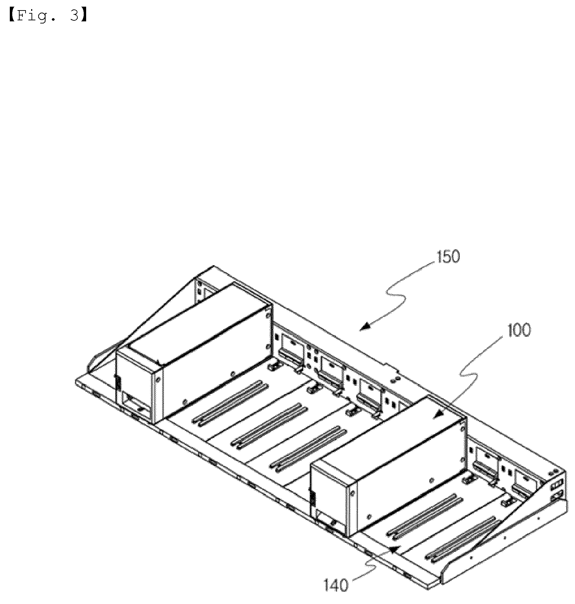

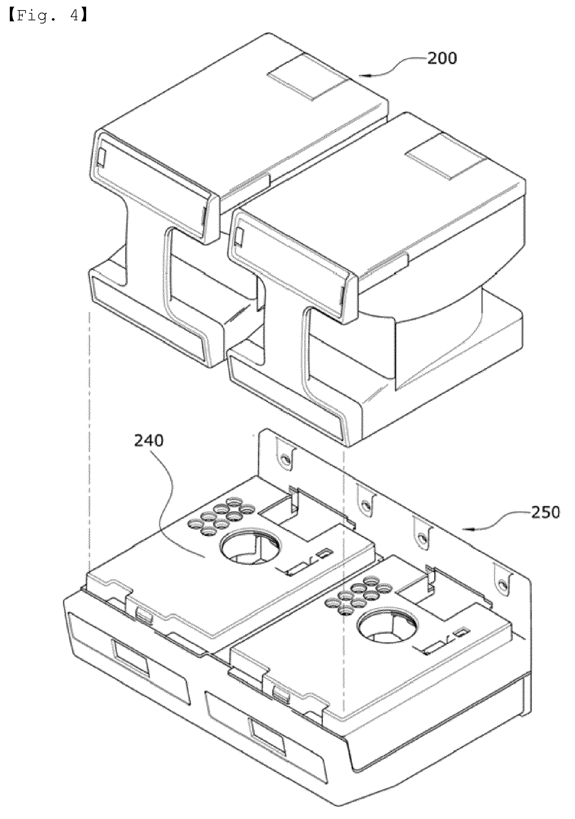

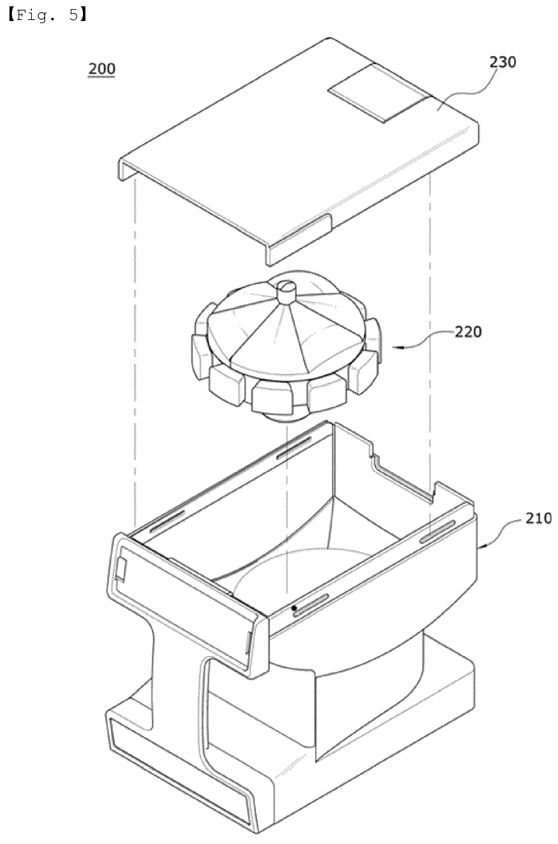

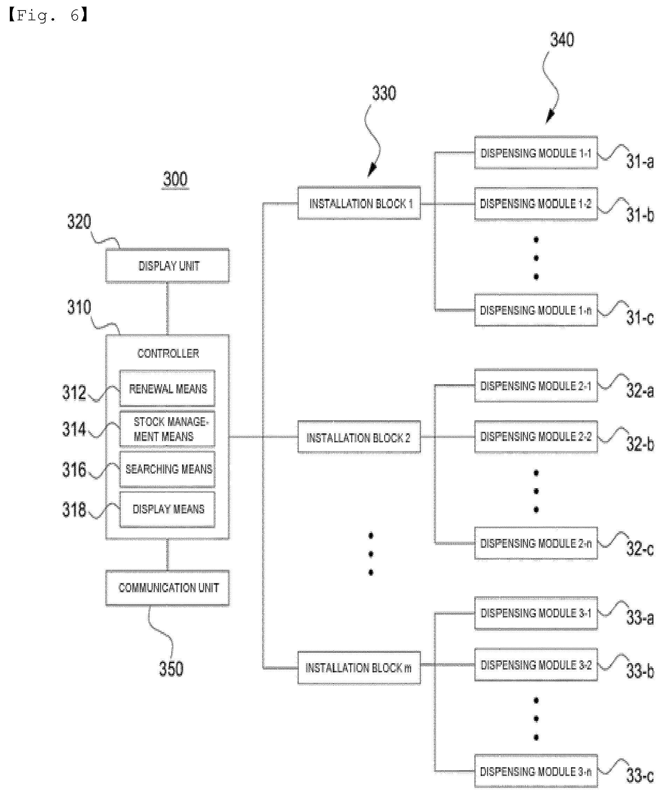

FIG. 2 is a perspective view schematically showing a drug dispensing device according to an embodiment of the present invention, FIG. 3 is a perspective view schematically showing an installation block and a dispensing module of FIG. 2, FIG. 4 is a perspective view schematically showing an installation block and a dispensing module according to another embodiment of the present invention, FIG. 5 is an exploded perspective view showing the dispensing module of FIG. 4, and FIG. 6 is a block diagram showing the construction of the drug dispensing device according to the embodiment of the present invention in detail.

Referring to FIG. 2, a drug dispensing device 300 according to an embodiment of the present invention may include an installation block 150 and a dispensing module 100. In the drug dispensing device 300 according to this embodiment, a plurality of installation blocks 150 may be provided at predetermined intervals in a vertical direction.

As shown in FIG. 3, each installation block 150 may be provided with a plurality of mounting units 140. The mounting units 140 may be arranged in a horizontal direction so as to provide a space in which to mount a plurality of dispensing modules 100. Each of the mounting units 140 may be provided with a terminal (not shown), through which each of the mounting units 140 is electrically connected to a corresponding one of the dispensing modules 100.

Various types of dispensing modules 100 may be provided. For example, various types of dispensing modules 100 may be provided such that the dispensing modules 100 can easily dispense drugs based on the physical properties of a container for containing drugs, the size of the container, or the shape of the container.

In addition, various types of dispensing modules 100 may be provided such that the dispensing modules 100 can easily dispense drugs contained in a fragile container, such as an ampoule, which is made of glass, or a vial, drugs that are too large to be dispensed together with other kinds of drugs through the same module, and drugs that are too small to be counted, such as tablets or pills.

Meanwhile, referring to FIG. 4, an installation block 250 may be provided with one or more mounting units 240.

Each dispensing module 200 may be mounted on a corresponding one of the mounting units 240. As shown in FIG. 5, each dispensing module 200 is configured to contain tablets or pills in an inner space defined by a body 210 and a cover 230 and to dispense the tablets or the pills one by one through a tablet or pill division and receiving part 220.

Meanwhile, in the drug dispensing device shown in FIGS. 2 and 3, each dispensing module 100 may also be referred to as a drug dispensing box or a drug dispensing block, and each installation block 150 may also be referred to as a cartridge. In addition, in the drug dispensing device shown in FIGS. 4 and 5, each dispensing module 200 may also be referred to as a division block, and each installation block 250 may also be referred to as a cartridge.

In this specification, therefore, the dispensing module 100 and the installation block 150 may be variously named. In the following description, however, a unit which is provided with a drug storage part for receiving drugs or containers, in which drugs are contained, and which is configured to dispense a predetermined number of drugs received in the drug storage part according to external electrical or mechanical control will be defined as a dispensing module, and a unit which is provided with a mechanical or electrical interface, through which a plurality of dispensing modules will be mounted to the unit, and which directly or indirectly controls the dispensation of drugs from the dispensing modules will be defined as an installation block.

Hereinafter, the construction of the drug dispensing device according to the embodiment of the present invention in detail will be described in more detail with reference to FIG. 6.

The drug dispensing device 300 according to the embodiment of the present invention may include a controller 310, a display unit 320, a communication unit 350, a plurality of installation blocks 330, and a plurality of dispensing modules 340.

As shown in FIG. 6, a plurality of installation blocks 330 may be electrically connected to the controller 310, and a plurality of dispensing modules 340 may be electrically connected to each of the installation blocks 330. For example, the controller 310 may control installation block 2 and may thus directly or indirectly control dispensing modules 2-1 to 2-n, which are connected to installation block 2.

The display unit 320 may be a means for providing information related to prescriptions and state and control information regarding the installation blocks 330 and the dispensing modules 340 such that a user can recognize the above-specified information. Specifically, the controller 310 may control at least one user interface UI for providing the above-specified information to the user to be displayed on the display unit 320.

Meanwhile, in a case in which a sensor for sensing touch operations (hereinafter, referred to as a 'touch sensor') is provided together with the display unit 320 so as to constitute a layered structure (hereinafter, referred to as a 'touchscreen'), the display unit 320 may also be used as an input device as well as an output device. The touch sensor may be configured, for example, in the form of a touch film, a touch sheet, or a touch pad.

The touch sensor may be configured to convert pressure applied to a specific region on the display unit 320 or the change of capacitance generated from a specific region on the display unit 320 into an electrical input signal. In addition, the touch sensor may be configured to sense touch pressure as well as touch position and touch area.

When the touch sensor senses a touch input, a corresponding signal(s) is transmitted to a touch control unit. The touch control unit processes the signal(s), and transmits corresponding data to the controller. As a result, the controller may determine which region on the display unit 320 has been touched.

The communication unit 350 is configured to transmit and receive various kinds of data to and from the server S. The communication unit 350 may include at least one module for allowing the server S and the drug dispensing device 300 to communicate with each other in a wireless fashion over the network N.

For example, the communication unit 350 may include a wireless Internet module, a near field communication module, and a position information module.

The wireless Internet module is a module for wireless Internet access. The wireless Internet module may be mounted inside or outside the drug dispensing device 300. Wireless LAN (WLAN), wireless fidelity (WiFi), wireless broadband (Wibro), world interoperability for microwave access (Wimax), or high speed downlink packet access (HSDPA) may be used as wireless Internet technology.

The near field communication module is a module for near field communication. Bluetooth, radio frequency identification (RFID), infrared data association (IrDA), ultra wideband (UWB), ZigBee, wireless HD (WiHD), or wireless gigabit alliance (WiGig) may be used as near field communication technology.

Specifically, the communication unit 350 may transmit various data related to prescriptions and data related to the state of the drug dispensing device 300 to the server S, and may store information received through the server S in the database DB. For example, the information received through the server S may include unique identification information assigned to each installation block 330 and identification information of the mounting units of each of the installation blocks 330 or interface units of each of the installation blocks 330, through which the dispensing modules 340 are electrically connected to each of the installation blocks 330. In mechanical terms, the dispensing modules 340 may be connected to each of the installation blocks 330 through the mounting units. In electrical terms, the dispensing modules 340 may be connected to each of the installation blocks 330 through the interface units. In the following description, the connection between the dispensing modules 340 and each of the installation blocks 330 through the mounting units will include an electrical connection between the dispensing modules 340 and each of the installation blocks 330 through the interface units for the convenience of description. In addition, in a case in which a specific dispensing module is mounted in a corresponding mounting unit of a specific installation block, identification information of the installation block and the mounting unit may include identification information of the dispensing module corresponding to the installation block and the mounting unit. The identification information of the dispensing module may further include identification information of drugs contained in the corresponding dispensing module.

Meanwhile, the controller 310 may include a renewal means 312, a stock management means 314, a searching means 316, and a display means 318.

The renewal means 312 may function to store and renew information to be stored in the database, either periodically or when a specific event occurs. When a dispensing module 340 is mounted in a specific mounting unit of an installation block 330 as described above, the renewal means 312 may store identification information of the installation block and the mounting unit and identification information of the dispensing module in the database. At this time, the renewal means 312 may also store identification information of drugs contained in the corresponding dispensing module.

The renewal means 312 may periodically renew the database. Alternatively, when it is notified through a renewal notification means provided at each installation block 330 that a new dispensing module 340 has been mounted and thus it is necessary to renew the database, the renewal means 312 may renew the database.

The stock management means 314 may determine which of the dispensing modules 340 needs to be refilled with drugs.

The stock management means 314 may determine which of the dispensing modules 340 needs to be refilled with drugs using various methods. Specifically, the stock management means 314 may subtract the number of drugs that have been dispensed from the total number of drugs contained in a specific dispensing module 340 to determine whether the dispensing module 340 needs to be refilled with drugs. In addition, the stock management means 314 may check the remaining number of drugs contained in the dispensing module 340 using various sensors. That is, the stock management method of the drug dispensing device 300 according to the embodiment of the present invention is not particularly restricted.

The searching means 316 may retrieve, from the database, information regarding the installation block and the mounting unit in which the dispensing module that has been determined to need to be refilled with drugs, as the result of determination of the stock management means 314, is located.

The display means 318 may display information related to at least one dispensing module retrieved from the database on the display unit 320.

Meanwhile, although not shown in the drawings, each installation block 330 may further include a memory, a mounting sensing unit, a recognition means, and a renewal notification means.

The memory is a storage mounted in each installation block 330. When a dispensing module is mounted in any one of the mounting units of a corresponding one of the installation blocks 330, the memory may store identification information of the corresponding mounting unit and identification information of the dispensing module mounted in the mounting unit. The identification information of the mounting unit and the identification information of the dispensing module may be transmitted to the renewal means 312, and may be stored in the database, as described above.

Each installation block 330 may be provided with interface units corresponding to the respective mounting units. When a specific dispensing module 340 is mounted in any one of the mounting units, the dispensing module 340 may transmit and receive control and data signals through the interface unit corresponding to the mounting unit.

The mounting sensing unit may sense whether a specific dispensing module 340 has been mounted in any one of the mounting units. The mounting sensing unit may be embodied using various electrical, mechanical, or optical sensors.

When the mounting sensing unit senses that the dispensing module 340 has been mounted in a specific mounting unit, the recognition means may acquire identification information of the corresponding dispensing module 340 and identification information of the mounting unit or the interface unit in which the corresponding dispensing module 340 is mounted, and may store the acquired information in the memory.

When the identification information of a series of dispensing modules and mounting units is stored in the memory by the recognition means, or when the mounting sensing unit senses that the dispensing module has been mounted in a specific mounting unit or a specific interface unit, the renewal notification means may transmit a signal related to whether to renew the information to the controller 310.

### 3. Construction of drug refilling station

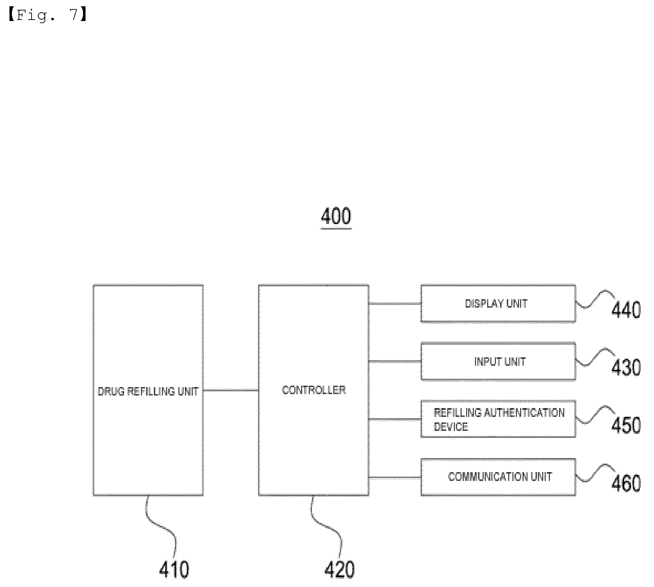

Hereinafter, the construction of a drug refilling station according to an embodiment of the present invention will be described in detail with reference to FIG. 7. FIG. 7 is a block diagram showing the construction of a drug refilling station according to an embodiment of the present invention in detail.

A drug refilling station 400 according to an embodiment of the present invention may include a drug refilling unit 410, a controller 420, an input unit 430, a display unit 440, a refilling authentication device 450, and a communication unit 460.

The drug refilling unit 410 may include at least one dispensing module that has been determined by the stock management means 314 to need to be refilled with drugs. Meanwhile, the drug refilling unit 410 may include a plurality of installation blocks, in which dispensing modules are mounted. Each of the installation blocks may be provided with a plurality of mounting units. The dispensing modules and the installation blocks provided in the drug refilling unit 410 may correspond in construction to the dispensing modules and the installation blocks included in the drug dispensing device 300, which as previously described with reference to FIGS. 2 to 5, and therefore a detailed description thereof will be omitted.

The input unit 430 allows a user to input data for controlling the operation of the drug refilling station 400. For example, the user may mount the dispensing module that has been determined to need to be refilled with drugs in the drug refilling unit 410, and the input unit 430 may be a means for allowing the user to input information related to the dispensing module that needs to be refilled with drugs.

Specifically, the information related to the dispensing module that needs to be refilled with drugs may include the name of drugs with which to refill the corresponding dispensing module, identification information of the corresponding dispensing module, and information regarding the quantity of drugs with which to refill the corresponding dispensing module.

Meanwhile, the input unit 430 may be embodied using a key pad, a dome switch, a (static pressure type or electrostatic type) touch pad, a jog wheel, a jog switch, or the like.

The display unit 440 may display information that is processed by the drug refilling station 400. In addition, the display unit 440 may be a means for providing control information necessary in order for the drug refilling unit 410 to perform the drug refilling operation such that the user can recognize the control information. That is, the display unit 440 may display at least one user interface UI for providing the user with information related to the dispensing module that needs to be refilled with drugs and refilling information regarding drugs with which the dispensing module has been refilled.

In a case in which a sensor for sensing touch operations (hereinafter, referred to as a 'touch sensor') is provided together with the display unit 440 so as to constitute a layered structure (hereinafter, referred to as a 'touchscreen'), the display unit 440 may also be used as an input device as well as an output device.

The touch sensor may be configured, for example, in the form of a touch film, a touch sheet, or a touch pad. The touch sensor may correspond to the touch sensor mentioned in the previous description of the drug dispensing device 300, and therefore a detailed description thereof will be omitted.

The refilling authentication device 450 may be a device for authenticating refilling information regarding a specific dispensing module that has been refilled with drugs by the drug refilling unit 410. In addition, it may be possible to confirm information related to the dispensing module mounted in the drug refilling unit using the refilling authentication device 450.

The refilling information may include at least identification information of drugs with which the corresponding dispensing module has been refilled and information regarding the quantity of the drugs.

The identification information of the drugs with which the corresponding dispensing module have been refilled may be read by various scanners, such as a barcode reader or an RFID reader, provided in the drug refilling station 400.

The identification information read by the scanner may be a unique identification code assigned to each dispensing module. The identification code may be a combination of predetermined alphabetic characters, numbers, or special symbols. Alternatively, the identification code may be a barcode constituted by combining a plurality of bars having different sizes. Alternatively, the identification code may be a two-dimensional QR code containing various kinds of information in a quadrangular lattice pattern. In addition, the identification information read by the scanner may include at least the identification number of the corresponding dispensing module, the name of drugs with which the corresponding dispensing module has been refilled, and the number of drugs with which the corresponding dispensing module has been refilled.

Meanwhile, in a case in which the refilling authentication device 450 is embodied using a RFID reader, an RFID tag for storing identification information of each dispensing module may be attached to the corresponding dispensing module. Consequently, the RFID reader may collect information stored in the RFID tag attached to each dispensing module through wireless transmission and reception to authenticate refilling information of drugs with which the corresponding dispensing module has been refilled.

In addition, the information regarding the quantity of drugs with which the dispensing module has been refilled may be read by a quantity measurement device provided in the drug refilling station. For example, the quantity measurement device may be configured to measure the weight of drugs with which a specific dispensing module has been refilled. For example, the quantity measurement device may be a scale mounted in the drug refilling station. Consequently, the controller 420 may estimate the quantity of drugs with which the specific dispensing module has been refilled based on the weight of drugs measured by the quantity measurement device.

The communication unit 450 may include at least one module for allowing the server S and the drug refilling station 400 to communicate with each other in a wireless fashion over the network N.

For example, the communication unit 460 may include a wireless Internet module, a near field communication module, and a position information module.

The wireless Internet module is a module for wireless Internet access. The wireless Internet module may be mounted inside or outside the drug refilling station 400. Wireless LAN (WLAN), wireless fidelity (WiFi), wireless broadband (Wibro), world interoperability for microwave access (Wimax), or high speed downlink packet access (HSDPA) may be used as wireless Internet technology.

The near field communication module is a module for near field communication. Bluetooth, radio frequency identification (RFID), infrared data association (IrDA), ultra wideband (UWB), ZigBee, wireless HD (WiHD), or wireless gigabit alliance (WiGig) may be used as near field communication technology.

Meanwhile, the drug refilling station 400 may transmit the refilling information authenticated by the refilling authentication device 450 to the server S through the communication unit 460. In addition, the refilling information received by the server S may be stored in the database DB.

The controller 420 may control the overall operations of the drug refilling station 400. The operation of refilling the drug refilling station will be described hereinafter in detail.

### 4. Operation of Refilling Drug Refilling Station

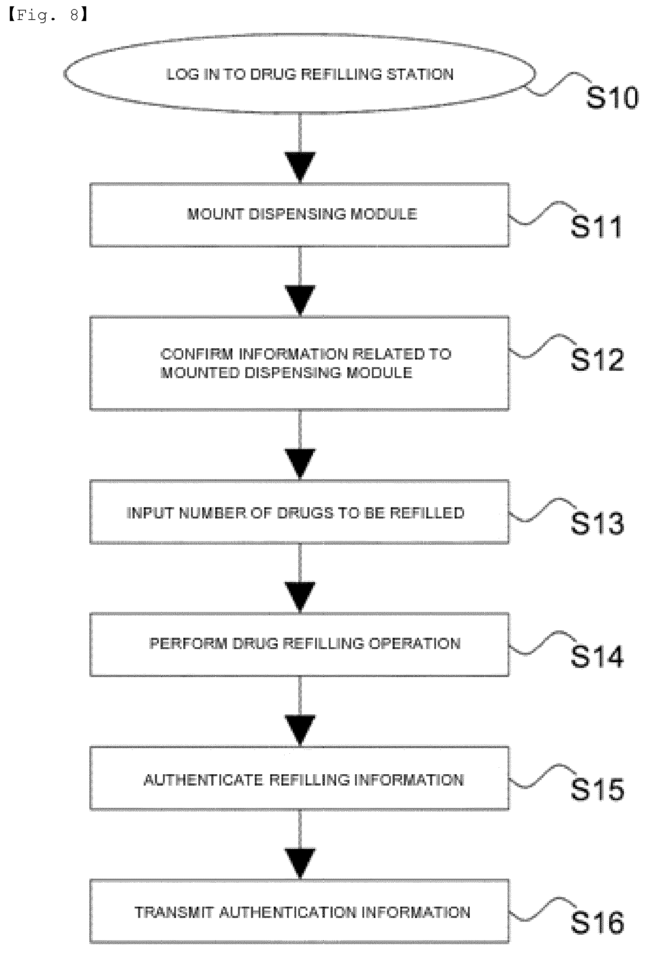

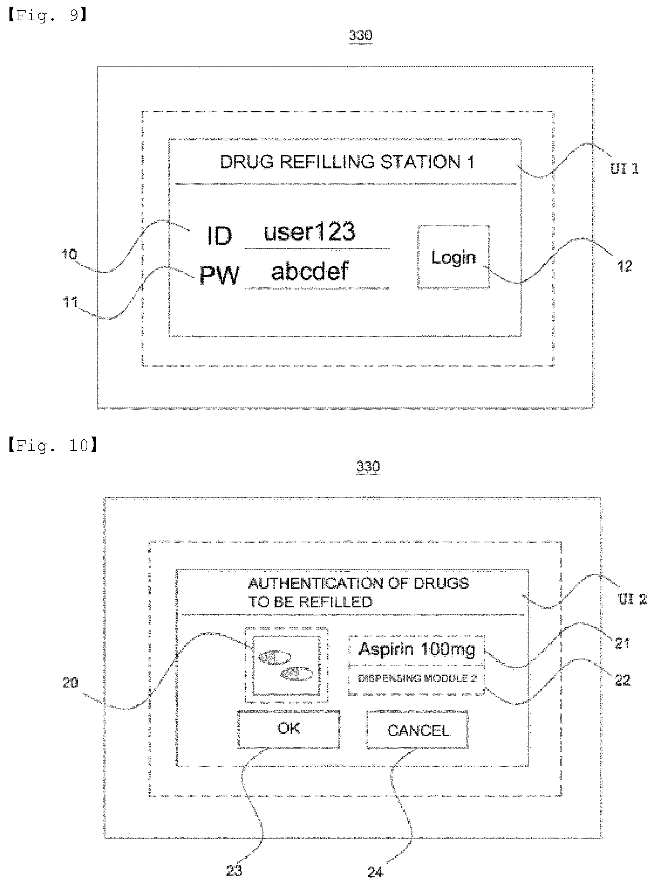

Hereinafter, a drug refilling method according to an embodiment of the present invention will be described in detail with reference to FIGS. 8 to 13. FIG. 8 is a flowchart illustrating a drug refilling method according to an embodiment of the present invention, and FIGS. 9 to 13 are views showing an example of a user interface for controlling the drug refilling station according to the embodiment of the present invention.

Referring to FIG. 8, the drug refilling method, performed by the drug refilling station 400 according to the embodiment of the present invention, may include a step of logging in the drug refilling station (S10), a step of mounting at least one dispensing module that needs to be refilled with drugs (S11), a step of scanning identification information of the mounted dispensing module (S12), a step of inputting the number of drugs with which the mounted dispensing module is to be refilled (S13), a step of performing a drug refilling operation according to input information (S14), a step of authenticating refilling information of the dispensing module that has been refilled with drugs (S15), and a step of transmitting authentication information (S16).

As shown in FIGS. 9 to 13, the controller 420 may provide various user interfaces UI for controlling the drug refilling station 400 on the input unit 430 or the display unit 440 of the drug refilling station 400.

Hereinafter, the respective steps will be described in detail on the assumption that various user interfaces UI are provided on the display unit 440 from the controller 420.

A doctor or a pharmacist (hereinafter, referred to as a 'user') may log in to the drug refilling station in order to refill the dispensing module that has been determined to need to be refilled from the stock management means 314 with drugs (S10).

The controller 420 may provide a first user interface for requesting identification information and/or authentication information of the user. The identification information and/or authentication information of the user may be unique identification information, such as an identification (ID) and password (PW), a registration number, a certificate of authentication and fingerprint information, face recognition information, voice recognition information, and iris recognition information, of the user that enables a specific user to have a right to access the drug refilling station 400. The identification information and/or authentication information of the user is provided to prevent an unauthorized user to perform the drug refilling operation, thereby preventing the occurrence of a medical incident.

For example, referring to FIG. 9, the controller 420 may provide a first user interface UI 1 for requesting an identification (ID) and password (PW) of a user in order to authenticate the user of drug refilling station 1. When an authorized user of drug refilling station 1 inputs "user123" 10, as the identification (ID), and "abcdef" 11, as the password (PW), and selects an image 12 corresponding to "Login," the controller 420 may grant the user a right to access drug refilling station 1. However, when an unauthorized user inputs incorrect identification (ID) or password (PW), the controller may deny the user a right to access drug refilling station 1.

The user who has been granted the right to access drug refilling station 1 from the controller 420 may mount at least one dispensing module that needs to be refilled with drugs in the drug refilling unit 410 (S11), and the controller 420 may confirm the information related to the mounted dispensing module (S12).

Specifically, the controller 420 may confirm the information related to the dispensing module mounted in the drug refilling unit 410 through the refilling authentication device 450. The information related to the dispensing module may include the name of drugs with which to refill the corresponding dispensing module, identification information of the dispensing module, and information regarding the quantity of drugs with which to refill the dispensing module.

For example, referring to FIG. 10, the controller 420 may provide a second user interface UI 2 including "dispensing module 2" 22, which is the identification number of the dispensing module confirmed by the refilling authentication device 450, "Aspirin 100mg" 21, which is the name of the drug corresponding to dispensing module 2, and an image 20 of the drug. Consequently, the user may confirm the information related to the mounted dispensing module, which has been confirmed by the refilling authentication device 450, and may select an image 23 corresponding to "OK" such that the next step of the drug refilling operation can be performed. On the other hand, the user may select an image 23 corresponding to "Cancel" to stop or interrupt the drug refilling operation.

The controller 420 may allow the user to input the number of drugs with which to refill the dispensing module mounted in the drug refilling unit 410 (S13).

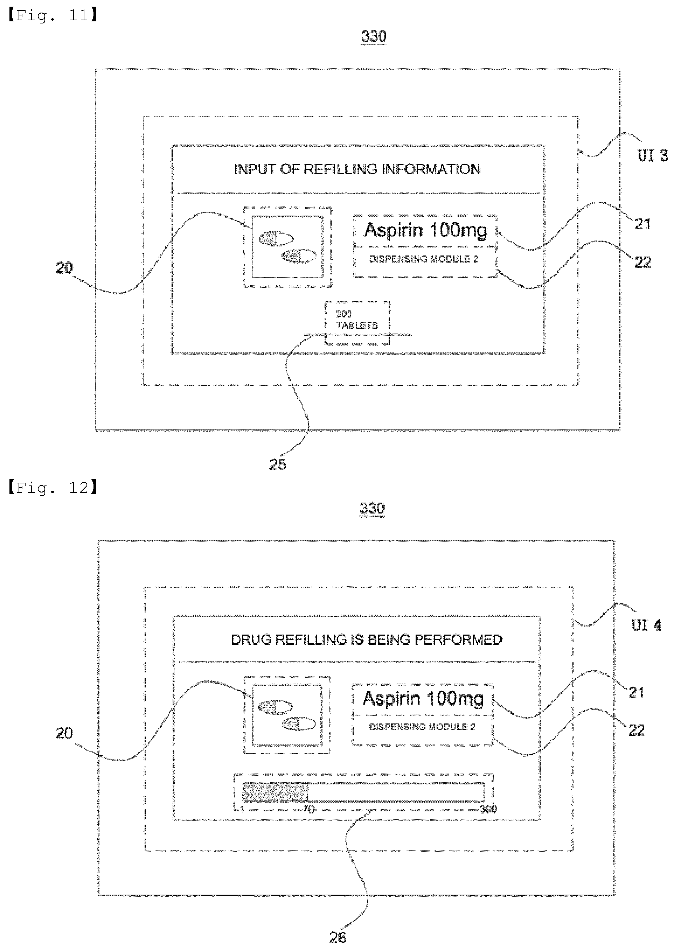

For example, referring to FIG. 11, the controller 420 may provide a third user interface UI 3 for allowing the user to input the number of drugs with which to refill the dispensing module. That is, when the user wishes to refill "dispensing module 2" 22 with 300 tablets of "Aspirin 100mg" 21, the user may input the number 25 corresponding to "300" using the input unit 430 or the touch panel of the display unit 440.

The controller 420 may control the operation of the drug refilling unit 410 such that the drug refilling unit 410 can perform the drug refilling operation according to information input by the user (S14). In addition, the controller 420 may provide a fourth user interface UI 4 for providing information regarding a state of progress of the drug refilling operation performed by the drug refilling unit 410. For example, referring to FIG. 12, a bar 26, indicating a state of progress of an operation of refilling the dispensing module with 300 tablets of "Aspirin 100mg," input by the user at step S13, may be used in order to display in real time that 70 tablets of "Aspirin 100mg" have been loaded. Furthermore, when the user requests that the drug refilling operation be stopped during the drug refilling operation, the controller 420 may control the drug refilling unit 410 to stop the drug refilling operation. Meanwhile, the user interface for displaying information regarding the state of progress of the drug refilling operation may be provided in various manners.

Upon completion of the drug refilling operation, the controller 420 may request the refilling authentication device 450 to authenticate refilling information of the dispensing module that has been refilled with drugs (S15).

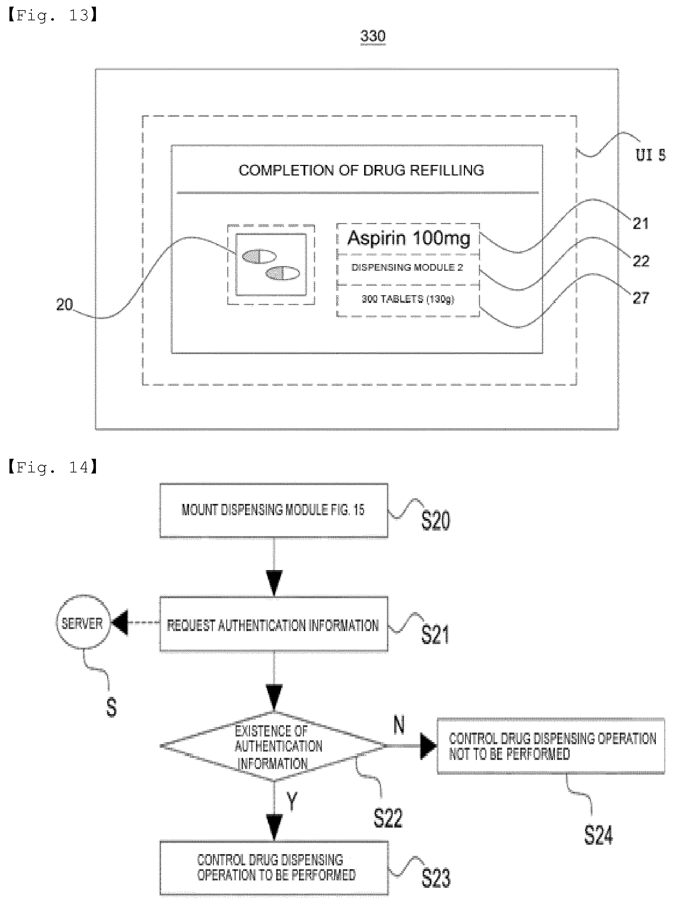

The controller 420 may acquire refilling information regarding "dispensing module 2" 22 that has been refilled with drugs from the refilling authentication device 450, and may provide a fifth user interface UI 5 for providing the acquired refilling information to the user. For example, referring to FIG. 13, the controller 420 may provide information, "300 tablets (130 g)" 27, regarding the number and/or weight of the drug, "Aspirin 100mg," with which "dispensing module 2" 22 has been refilled, through the fifth user interface UI 5.

The controller 420 may transmit authentication information, authenticated by the refilling authentication device 450, to the server S (S16). The server S may store the received authentication information in the database DB.

### 5. Control of Drug Dispensing Operation

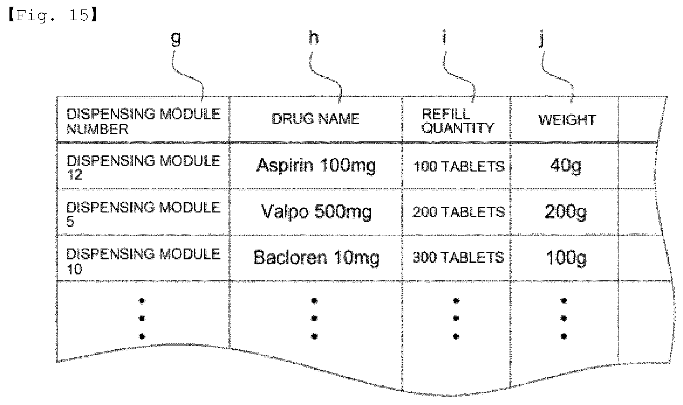

Hereinafter, a method of controlling the drug dispensing system according to the embodiment of the present invention will be described in detail. FIG. 14 is a flowchart illustrating a method of controlling the drug dispensing system according to the embodiment of the present invention, and FIG. 15 is a table illustrating information stored in the database of the drug dispensing system according to the embodiment of the present invention.

In addition, hereinafter, the method of controlling the drug dispensing system will be described on the assumption that "dispensing module 2" and/or "dispensing module 12," which has been determined to need to be refilled by the stock management means 314, has been refilled with drugs by the drug refilling station 400, for the convenience of description.

Referring to FIG. 14, the method of controlling the drug dispensing system 100 according to the embodiment of the present invention may include a step of mounting a dispensing module that has been refilled in an installation block (S20), a step of requesting authentication information (S21), a step of confirming the authentication information (S22), and a step of performing a drug dispensing operation (S23) or a step of stopping the drug dispensing operation (S24).

When at least one dispensing module that has been refilled with drugs is mounted in a specific mounting unit of the drug dispensing device 300 (S20), the controller 320 may request authentication information from the server S in order to determine whether the mounted dispensing module has been refilled with drugs by the drug refilling station 400 (S21).

The authentication information may be refilling information regarding the first dispensing module authenticated by the drug refilling station 400 as previously described. The refilling information may include at least identification information of drugs with which the corresponding dispensing module has been refilled and information regarding the quantity of the drugs. That is, referring to FIG. 15, the database DB may store information regarding dispensing module number 30, drug name 31, refill quantity 32, and weight 33.

The communication unit 350 may transmit a request for the authentication information to the server S, and the server S may check the authentication information stored in the database DB (S22). That is, upon receiving the request for the authentication information from the controller 320, the server S may determine whether authentication information corresponding to the request of the authentication information is stored in the database DB.

For example, the server S may receive the request of the authentication information for authenticating the refilling information regarding 'dispensing module 2' from the controller 320, and may determine whether refilling information regarding 'dispensing module 2' is stored in the database DB. Upon determining that no refilling information regarding 'dispensing module 2' exists in the table of FIG. 15 as the result of confirming the authentication information, the server S may transmit a response signal indicating that no refilling information exists. Consequently, the controller 320 may control the drug dispensing device 300 not to perform the drug dispensing operation (S24).

In another example, the server S may receive a request for authentication information for authenticating the refilling information regarding 'dispensing module 12' from the controller 320, and may determine whether the refilling information regarding 'dispensing module 12' is stored in the database DB. Upon determining that refilling information regarding 'dispensing module 12' exists in the table of FIG. 15 as the result of confirming the authentication information, the server S may transmit a response signal indicating that refilling information exists. Consequently, the controller 320 may control the drug dispensing device 300 to perform the drug dispensing operation (S23).

As is apparent from the above description, in a drug dispensing system according to the present invention, it is not necessary to install a plurality of computers corresponding to a plurality of drug refilling stations, and it is possible to manage a drug refilling operation using a single client PC, thereby improving the efficiency of drug collection. Furthermore, it is possible to construct a drug dispensing system including a plurality of drug refilling stations without limitations as to a position at which the drug dispensing system is installed.

In addition, in the drug dispensing system according to the present invention, only a dispensing module that has been authenticated by a drug refilling station assigned to the drug dispensing system in advance is controlled to perform a drug dispensing operation, thereby preventing the occurrence of a medical incident.

It will be apparent that, although the preferred embodiments have been shown and described above, the present invention is not limited to the above-described specific embodiments, and various modifications and variations can be made by those skilled in the art without departing from the technical idea of the present invention. In addition, the embodiments described in this specification are not restrictively applied, and all or some of the embodiments may be selectively combined to provide various modifications.

## Claims

1. A drug dispensing system comprising:
at least one drug dispensing device comprising a plurality of dispensing modules for receiving and dispensing drugs, a plurality of installation blocks for allowing the dispensing modules to be mounted therein, and a controller for controlling dispensing of drugs from the dispensing modules;
a plurality of drug refilling stations, each drug refilling station comprising an input unit for allowing information related to a first dispensing module that needs to be refilled with drug, selected from among the dispensing modules, to be input, a drug refilling unit for performing a drug refilling operation necessary to refill the first dispensing module with corresponding drugs according to the information related to the first dispensing module, a refilling authentication device for authenticating refilling information regarding the drugs refilled by the drug refilling unit, and a communication unit for transmitting authentication information regarding the first dispensing module confirmed by the refilling authentication device;
a server for receiving the authentication information regarding the first dispensing module from the communication unit; and
a database for storing the authentication information regarding the first dispensing module received from the server, wherein
when a second dispensing module is mounted in the installation block, the controller requests authentication information regarding the second dispensing module from the server, and, upon determining that no authentication information regarding the second dispensing module exists in the database, controls a drug dispensing operation not to be performed.

2. The drug dispensing system according to claim 1, wherein
the drug refilling stations are assigned to the server, and
the database stores the authentication information regarding the first dispensing module authenticated by at least one selected from among the drug refilling stations assigned to the server, received from the server.

3. The drug dispensing system according to claim 1, wherein the controller comprises a stock management means for determining which of the dispensing modules needs to be refilled with drugs.

4. The drug dispensing system according to claim 3, wherein at least one dispensing module that has been determined to need to be refilled with drugs by the stock management means is mounted in the drug refilling unit.

5. The drug dispensing system according to claim 1, wherein the information related to the dispensing module comprises at least a name of drugs with which to refill the dispensing module, identification information of the dispensing module, and information regarding a quantity of drugs with which to refill the dispensing module.

6. The drug dispensing system according to claim 1, wherein the refilling authentication device is a barcode reader for reading the refilling information.

7. The drug dispensing system according to claim 1, wherein the refilling authentication device is a radio frequency identification (RFID) reader for reading the refilling information.

8. The drug dispensing system according to claim 1, wherein the refilling authentication device comprises a quantity measurement device for measuring a quantity of drugs with which the dispensing module has been refilled.

9. The drug dispensing system according to claim 8, wherein the quantity of drugs with which the dispensing module has been refilled is estimated based on a weight of the drugs measured by the quantity measurement device.

10. The drug dispensing system according to claim 1, wherein the refilling information confirmed by the refilling authentication device comprises at least identification information of the drugs with which the dispensing module has been refilled and information regarding a quantity of the drugs.

11. The drug dispensing system according to claim 1, wherein the authentication information comprises at least a name of drugs with which the dispensing module has been refilled, identification information of the dispensing module, and information regarding a quantity of drugs with which the dispensing module has been refilled.

12. The drug dispensing system according to claim 1, wherein, when the first dispensing module is mounted in the installation block, and upon determining that the authentication information regarding the first dispensing module exists in the database, the controller controls the drug dispensing operation to be performed.

13. A method of controlling a drug dispensing system, the method comprising:
mounting a dispensing module that has been refilled;
transmitting a request of authentication information regarding the mounted dispensing module;
receiving a response signal indicating whether the authentication information regarding the mounted dispensing module exists in response to the request of the authentication information; and
when the response signal indicates that the authentication information regarding the refilled dispensing module exists, controlling the refilled dispensing module to perform a drug dispensing operation.

14. The method according to claim 13, further comprising, when the response signal indicates that no authentication information regarding the refilled dispensing module exists, controlling the refilled dispensing module so as not to perform the drug dispensing operation.
